# EUROPEAN PATENT SPECIFICATION

(11) **EP 1 407 763 B1**
(45) Date of publication and mention of the grant of the patent: **14.06.2006**
(21) Application number: 03256101.1
(22) Date of filing: 29.09.2003
(51) Int. Cl.: A61K 8/368, A61K 8/44, A61K 8/46, A61K 8/97, A61Q 19/00, A61Q 19/10

(54) **Compositions containing a cosmetically active organic acid and a legume product**
Zusammensetzungen enthaltend eine kosmetisch-aktive organische Säure und ein Gemüseprodukt
Compositions contenant une acide organique à activité cosmétique et un produit légume

(30) Priority: 30.09.2002 US 261522
(43) Date of publication of application: 14.04.2004
(73) Proprietor: JOHNSON & JOHNSON CONSUMER COMPANIES, INC., Skillman, NJ 08858 (US)
(72) Inventor: Wu, Jeffrey M., Warrington PA 18976 (US); Liu, Jue-Chen, Belle Mead NJ 08502 (US); Ho, Kie, Princeton NJ 08540 (US)
(74) Representative: Fisher, Adrian John

(56) References cited:
- EP-A- 1 210 946
- EP-A- 1 236 465
- DE-A- 19 818 849

## Description

### FIELD OF THE INVENTION

The present invention relates to a cleansing composition containing a legume product.

### BACKGROUND OF THE INVENTION

Liquid personal cleansing products have gained popularity in the world. Desirable cleansing compositions usually not only provide good cleansing and lathering properties, but also do not irritate the skin. Recently, skin benefit agents such as cosmetically active organic acids have been added to such products. However, because of the surfactants present in the cleansing products, it has often been difficult to obtain the desired deposition of the benefit agent onto the skin.

Applicants have surprisingly found that the addition or a legume product to cleansing compositions enhances the deposition of the benefit agent contained therein.

### SUMMARY OF THE INVENTION

In one aspect, the present invention relates to a liquid cleansing composition containing (i) a surfactant, (ii) a legume product, and (iii) a cosmetically active agent.

In another aspect, the invention features a method of cleansing the skin, hair, or nail by applying to the skin a composition containing (i) a surfactant, (ii) a legume product, and (iii) cosmetically active agent.

The present invention also features a method of enhancing the deposition of a cosmetically active agent from a composition onto the skin by adding a legume product to the composition, wherein the composition contains a surfactant.

In one embodiment, the cosmetically active agent is a cosmetically active organic acid or a cosmetically acceptable salt or ester thereof.

Other features and advantages of the present invention will be apparent from the detailed description of the invention and from the claims

### DETAILED DESCRIPTION OF THE INVENTION

It is believed that one skilled in the art can, based upon the description herein, utilize the present invention to its fullest extent. The following specific embodiments are to be construed as merely illustrative, and not limitative of the remainder of the disclosure in any way whatsoever.

Unless defined otherwise, all technical and scientific terms used herein have the same meaning as commonly understood by one of ordinary skill in the art to which the invention belongs.

As used herein, all percentages are by weight unless otherwise specified.

As used herein, "trypsin inhibitory activity" means the ability of the legume product at a concentration of 0.1% (w/w) to inhibit the activity of the protease trypsin, as measured by the assay set forth below in Example 1 of European Patent Application No. 1,236,465.

In one embodiment, the legume products of the present invention have a trypsin inhibitory activity of at least about 15%. In a further embodiment, the legume products of the present invention have a trypsin inhibitory activity of at least about 25%, such as at least about 50%.

As used herein, "microbial content" means the amount of bacteria, fungi, and yeast present in the legume product. Examples of means to measure microbial content include, but are not limited to, the AOAC 986.23 Method as set forth in "Official Methods of Analysis of AOAC International," edited by Patricia Cunniff, Sixteenth Edition, 5^{th} Revision, 1999 (AOAC International) or the USP Method as set forth in "Official Compendia of Standards, USP 24 USP/NF 19", United States Pharmacopeial Convention, Inc., 2000 (Board of Trustees, United States Pharmacopeial Convention, Inc.).

"Objectionable microbial content" means the amount of bacteria, fungi, and yeast present in the legume product that are harmful to humans, including but not limited to coliform, E. Coli, Salmonella, thermophilic spores, Bacillus, Enterococcus, Staphylococcus, fecal streptococcus, and those listed in "Disinfection, sterilization, and preservation" 4th edition, Seymour S. Block, pp. 887-888 (1991, Lea & Febiger, Malvern, PA).

As used herein, "topical application" means directly laying on or spreading on outer skin using, e.g., by use of the hands or an applicator such as a wipe, puff, roller, or spray.

As used herein, "cosmetically-acceptable" means that the product(s) or compound(s) which the term describes are suitable for use in contact with tissues (e.g., the skin) without undue toxicity, incompatibility, instability, irritation, allergic response, and the like. This term is not intended to limit the ingredient/product to which it describes for use solely as a cosmetic (e.g., the ingredient/product may be used as a pharmaceutical).

As used herein, "topical carrier" means one or more compatible solid or liquid filler diluents that are suitable for topical administration to a mammal. Examples of topical carriers include, but are not limited to, water, waxes, oils, emollients, emulsifiers, thickening agents, gelling agents, and mixtures thereof.

As used herein, "regulating the firmness" means the enhancing of the firmness or elasticity, preventing the loss of firmness or elasticity, or preventing or treating sagging, lax and loose skin, hair, or nails. The firmness or elasticity of the skin can be measured by use of a cutometer. See Handbook of Non-Invasive Methods and the Skin, eds. J. Serup & G. Jemec, Chapter 14.3 (1995). The loss of skin elasticity or firmness may be a result of a number of factors, including but not limited to aging, external aggressions, or the result of an application of a cosmetic to the skin, hair, or nails.

As used herein, "regulating the tone" means the lightening and/or darkening of the appearance of the skin, hair, or nails (e.g., lightening pigmented lesions, darkening skin sallowness, and/or evening the color of the skin).

As used herein, "delaying or reducing nail growth" means the delaying or reducing the growth rate of the nail.

As used herein, "delaying or reducing hair growth" means the delaying or reducing the growth rate of the hair and/or width of hair shaft, including, but not limited to, the reducing the visibility or appearance of hair (e.g., hair on the arms, legs, and face).

As used herein, "cleansing" means the removal of dirt and/or oil from the skin, hair, or nail surface. What is meant by a "cleansing composition" is a composition that is to be applied and later removed from the skin, hair or nail (e.g., by rinsing or wiping) for the purpose of cleansing the same. Examples of cleaning compositions include, but are not limited to, shampoos, skin cleansing bars, and liquid skin cleansers.

As used herein, "regulating the texture" means the smoothing of the surface of the skin, hair, or nail to remove either bumps or crevasses on the surface, including, but mot limited to, smoothing or evening the appearance of the skin.

As used herein, "regulating wrinkles in skin" means preventing, retarding, arresting, or reversing the process of wrinkle or fine line formation in skin, including, but not limited to, reducing the visibility or appearance of wrinkles.

As used herein, "treatment of external aggressions" means the reduction or prevention of the damage from external aggressions in skin, hair, or nails caused by physical contact, chemical contact, or temperature. Examples of external aggressions include, but are not limited to, damage to the skin, hair, and nails from: the use of chemicals such as cleansers (e.g., skin and hair cleansers containing surfactants) and make-up; physical contact such as damage caused by shaving and cutting; and the environment such as chemical damage from ozone, exhaust, pollution, chlorine and compounds containing chlorine, and cigarette smoke, mechanical damage caused by the wind, damage caused by cold or hot temperature, and damage caused by UV light (e.g., sun damage from the sunlight or non-natural sources such as UV lamps and solar simulators). Effects of external aggressions on the skin, nails, and skin include, but are not limited to, oxidative and/or nitrosative damage to and modifications on lipids, carbohydrates, peptides, proteins, nucleic acids, and vitamins. Effects of external aggressions also include, but are not limited to, loss of cell viability, loss or alteration of cell functions, and changes in gene and/or protein expression.

As used herein, "safe and effective amount" means an amount of compound or composition (e.g., the legume product) sufficient to induce a positive modification in the condition to be regulated or treated, but low enough to avoid serious side effects. The safe and effective amount of the compound or composition will vary with the particular condition being treated, the age and physical condition of the end user, the severity of the condition being treated/prevented, the duration of the treatment, the nature of other treatments, the specific compound or product/composition employed, the particular cosmetically-acceptable carrier utilized, and like factors.

### Legume Product

What is meant by a "legume product" is a substance derived from a legume fruit. A legume is a plant from the family Leguminosae, which has a dehiscent fruit such as a bean, pea, or lentil. Examples of legumes, include but are not limited to, beans such as soybeans, lentil beans, peas, and peanuts.

The legume product may contain the entire legume fruit (e.g., the legume fruit ground into a powder) or only a portion of the legume (e.g., an extract of the legume). The legume product may be in the form of a fluid (e.g., legume fruit milk, which is a mixture of the legume fruit powder and water) or a solid (e.g., legume fruits powders or legume fruit milk powder such as soy milk powder). When in the form of a fluid, the term "legume product" refers to the solid constituents of the fluid derived from the legume.

The compositions of the present invention comprise a safe and effective amount of the legume product (e.g., soy product). In one embodiment, the composition contains from about 0.01% to about 50%, such as from about 0.1% to about 20% or from about 1% to about 10%, by weight, of the legume product (e.g., a soy product).

### Soy Product

What is meant by a "Soy Product" is a substance derived from the soybean. The soy product may contain only a portion of the soybean (e.g., an extract of the soybean such as a lipid reduced soybean powder or filtered soymilk) or may contain the entire soybean (e.g., a ground powder of the legume). The soy product may be in the form of a fluid (e.g., soymilk) or a solid (e.g., a soybean powder or soymilk powder). When in the form of a fluid, the term "soy product" refers to the solid constituents of the fluid that are derived from the soybean.

In one embodiment, the soy product is soybean powder. Soybean powder may be made by grinding dry soybeans. In one embodiment, the soybean powder has a average particle size of less than about 100 micrometers such as less than about 10 micrometers. In one embodiment, the soybean powder has a moisture content of less than about 10% such as less than about 5%. In one embodiment, the soybean powder is lyophilized.

In one embodiment, the soy product is soymilk or soymilk powder. Soymilk is a combination of solids derived from soybeans and water, the mixture of which has some or all of the insoluble constituents filtered off. Soymilk powder is evaporated soymilk, which in one embodiment, is in a lyophilized or spray-dried form. Procedures for manufacturing soymilk include, but are not limited to, the following three procedures. First, soymilk may be made by placing soybeans into water to allow them to absorb the water. The swelled beans are then ground and additional water is then added. The mixture may then filtered to remove any insoluble residue. Second, soymilk may also be prepared from soybean powder. Soybean powder is thoroughly mixed with water (e.g., for at least one hour), which may then be followed by a filtration process to remove insoluble residues. Third, soymilk can also be reconstituted from soymilk powder by adding water. In one embodiment, soymilk comprises from between about 1% to about 50%, by weight (e.g., from about 5% to about 20%, by weight) of solids from the soybean.

### Anti-microbial Treatment of Legume Product

As discussed above, the surface of legume fruits often contain high levels of microorganisms. Thus, prior to use by humans, the legume product needs to be treated to reduce or eliminate such microorganisms.

In one embodiment, the legume products of the present invention have a total microbial content of less than about 10,000 colony-forming units ("cfu") per gram. In a further embodiment, the soy products of the present invention have a microbial content of less than about 1,000 cfu per gram (such as less than about 100 cfu per gram) of the legume product.

In one embodiment, the legume products of the present invention have a total objectionable microbial content of less than 300 cfu per gram such as less than 150 cfu per gram. In a further embodiment, the legume products of the present invention have an undetectable amount of any objectionable microbials for at least one gram (e.g., at least ten grams) of legume product.

In one embodiment, the legume product is exposed to gamma irradiation. In a further embodiment, the legume product is exposed to between about 2 to about 30 kGy of gamma irradiation, such as between about 5 and about 10 kGy of gamma irradiation. Applicants have unexpectedly found that such treatment reduces the microbial content of the legume product, while maintaining its biological activity (e.g., serine protease inhibitory activity). Applicants have also found that treatment of legume products with gamma irradiation maintains the cosmetic elegance of the legume product, such as maintained its natural colors and did not induce significant malodors.

Other anti-microbial processes that also maintain the protease inhibitory activity of the legume product that can be practiced alone or in combination with gamma irradiation, include, but are not limited to, exposure to x-rays, high energy electron or proton beams, ultraviolet radiation, hydrostatic pressure, and addition of chemical agents possessing antimicrobial.activity, and combinations thereof. A complete list of methods for microbial content reduction is set forth in "Disinfection, sterilization, and preservation" 4th edition, Seymour S. Block, pp. 887-888 (1991, Lea & Febiger, Malvern, PA).

### Topical Compositions

The topical compositions useful in the present invention involve formulations suitable for topical application to skin. In one embodiment, the composition comprises the soy product and a cosmetically-acceptable topical carrier. In one embodiment, the cosmetically-acceptable topical carrier is from about 50% to about 99.99%, by weight, of the composition (e.g., from about 80% to about 95%, by weight, of the composition.

The compositions may be made into a wide variety of product types that include but are not limited to solid and liquid compositions such as lotions, creams, gels, sticks, sprays, shaving creams, ointments, cleansing liquid washes and solid bars, shampoos, pastes, powders, mousses, shaving creams, and wipes. These product types may comprise several types of cosmetically acceptable topical carriers including, but not limited to solutions, emulsions (e.g., microemulsions and nanoemulsions), gels, solids and liposomes. The following are non-limitative examples of such carriers. Other carriers can be formulated by those of ordinary skill in the art.

The topical compositions useful in the present invention can be formulated as solutions. Solutions typically include an aqueous solvent (e.g., from about 50% to about 99.99% or from about 90% to about 99% of a cosmetically acceptable aqueous solvent).

Topical compositions useful in the subject invention may be formulated as a solution comprising an emollient. Such compositions preferably contain from about 2% to about 50% of an emollient(s). As used herein, "emollients" refer to materials used for the prevention or relief of dryness, as well as for the protection of the skin. A wide variety of suitable emollients are known and may be used herein. See International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7^{th} Edition, 1997) (hereinafter "INCI Handbook") contains numerous examples of suitable materials.

A lotion can be made from such a solution. Lotions typically comprise from about 1% to about 20% (e.g., from about 5% to about 10%) of an emollient(s) and from about 50% to about 90% (e.g., from about 60% to about 80%) of water.

Another type of product that may be formulated from a solution is a cream. A cream typically comprises from about 5% to about 50% (e.g., from about 10% to about 20%) of an emollient(s) and from about 45% to about 85% (e.g., from about 50% to about 75%) of water.

Yet another type of product that may be formulated from a solution is an ointment. An ointment may comprise a simple base of animal or vegetable oils or semi-solid hydrocarbons. An ointment may comprise from about 2% to about 10% of an emollient(s) plus from about 0.1% to about 2% of a thickening agent(s). A more complete disclosure of thickening agents or viscosity increasing agents useful herein can be found in the INCI Handbook pp. 1693-1697.

The topical compositions useful in the present invention formulated as emulsions. If the carrier is an emulsion, from about 1% to about 10% (e.g., from about 2% to about 5%) of the carrier comprises an emulsifier(s). Emulsifiers may be nonionic, anionic or cationic. Suitable emulsifiers are disclosed in, for example, INCI Handbook, pp.1673-1686.

Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type are also useful in the subject invention. In general, such single or multiphase emulsions contain water, emollients, and emulsifiers as essential ingredients.

The topical compositions of this invention can also be formulated as a gel (e.g., an aqueous gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprises between about 0.1% and 5%, by weight, of such gelling agents.

The topical compositions of the present invention can also be formulated into a solid formulation (e.g., a wax-based stick, soap bar composition, powder, or a wipe containing powder).

The topical compositions useful in the subject invention may contain, in addition to the aforementioned components, a wide variety of additional oil-soluble materials and/or water-soluble materials conventionally used in compositions for use on skin, hair, and nails at their art-established levels.

### Surfactants

In one embodiment, the composition contains one or more surfactants. In one embodiment, the composition contains a lathering surfactant. What is meant by a lathering surfactant is a surfactant that generates lather when combined with water and mechanically agitated. In one embodiment, the lathering surfactant has an initial foam height reading of at least 20 mm, such as at least 50 mm, in the Standard Test Method for Foaming Properties of Surface-Active Agents D1173-53 Set forth in the ASTM Annual Book of ASTM Standards 1001 Section 15 Volume 15.04 (using a concentration of 5 grams per liter, temperature of 49°C, and water hardness of 8 grains per gallon). Examples of lathering surfactants include, but are not limited to, anionic, nonionic, cationic, and amphoteric lathering surfactants.

Nonlimiting examples of anionic lathering surfactants include those selected from the group consisting of sarcosinates, sulfates, isethionates, taurates, phosphates, lactylates, and glutamates. Specific examples include, but are not limited to, those selected from the group consisting of sodium lauryl sulfate, ammonium lauryl sulfate, ammonium laureth sulfate, sodium laureth sulfate, sodium trideceth sulfate, ammonium cetyl sulfate, sodium cetyl sulfate, ammonium cocoyl isethionate, sodium lauroyl isethionate, sodium lauroyl lactylate, triethanolamine lauroyl lactylate, sodium caproyl lactylate, sodium lauroyl sarcosinate, sodium myristoyl sarcosinate, sodium cocoyl sarcosinate, sodium lauroyl methyl taurate, sodium cocoyl methyl taurate, sodium lauroyl glutamate, sodium myristoyl glutamate, and sodium cocoyl glutamate and mixtures thereof

Nonlimiting examples of nonionic lathering surfactants include those selected from the group consisting of alkyl glucosides, alkyl polyglucosides, polyhydroxy fatty acid amides, alkoxylated fatty acid esters, lathering sucrose esters, amine oxides, and mixtures thereof. Specific examples include, but are not limited to, nonionic surfactants to those selected form the group consisting of C8-C14 glucose amides, C8- C14 alkyl polyglucosides, sucrose cocoate, sucrose laurate, lauramine oxide, cocoamine oxide, and mixtures thereof.

Nonlimiting examples of amphoteric lathering surfactants (which also includes zwitterionic lathering surfactants are those selected from the group consisting of betaines, sultaines, hydroxysultaines, alkyliminoacetates, iminodialkanoates, aminoalkanoates, and mixtures thereof.

Nonlimiting examples of amphoteric surfactants of the present invention include disodium lauroamphodiacetate, sodium lauroamphoacetate, cetyl dimethyl betaine, cocoamidopropyl betaine, cocoamidopropyl hydroxy sultaine, and mixtures thereof.

### Cosmetically Active Organic Acid

In one embodiment, the composition comprises a cosmetically active organic acid or a cosmetically acceptable salt or ester thereof. What is meant by a "cosmetically active organic acid" is a compound that has a cosmetic or therapeutic effect on the skin, hair, or nails. In one embodiment, the cosmetically active organic acid has a pKa of less then about 4. Examples of cosmetically active organic acid include, but are noyt limited to, hydroxy acids such as alpha-hydroxy acids, beta-hydroxy acids, poly-hydroxy acids, lipoic acid, dihydroxy lipoic acid, amino acids such a proline and tyrosine, peptides, retinoic acid, fatty acids such as linoleic acid, linolenic acid, and oleic acid, lactobionic acid, aspirin, ibuprofen, and ascorbic acid. Examples of alpha hydroxy acids include, but are not limited to, glycolic acid, lactic acid, malic acid, citric acid, and tartaric acid. Nonlimiting examples of beta hydroxy acids include salicylic acid. Other hydroxy acids are disclosed in European Patent Application No. 273,202.

In one embodiment, the composition contains from about 0.001% to about 10%, from about 0.5% to about 2%, of the cosmetically active organic acid or salt or ester thereof.

### Additional Cosmetically Active Agents

In one embodiment, the topical composition further comprises another cosmetically active agent in addition to the legume product. What is meant by a "cosmetically active agent" is a compound that has a cosmetic or therapeutic effect on the skin, hair, or nails, e.g., lightening agents, darkening agents such as self-tanning agents, anti-acne agents, shine control agents, anti-microbial agents, anti-inflammatory agents, anti-mycotic agents, ant-dandruff agents, anti-parasite agents, external analgesics, sunscreens, photoprotectors, antioxidants, keratolytic agents, moisturizers, nutrients, vitamins, energy enhancers, anti-perspiration agents, astringents, deodorants, hair removers, firming agents, anti-callous agents, and agents for hair, nail, and/or skin conditioning.

In one embodiment, the agent is selected from, but not limited to, the group consisting of benzoyl peroxide, sulfur, resorcinol, D-panthenol, hydroquinone, octyl methoxycinnimate, titanium dioxide, octyl salicylate, homosalate, avobenzone, polyphenolics, carotenoids, free radical scavengers, spin traps, retinoids such as retinol and retinyl palmitate, ceramides, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, peptides such as those disclosed in PCT Patent Application WO00/15188, vitamins, acetyl-coenzyme A, niacin, riboflavin, thiamin, ribose, azoles such as miconazole and ketoconazole, zinc pyrithione, coal tar, triclosan, electron transporters such as NADH and FADH2, and other botanical extracts such as aloe vera, and derivatives and mixtures thereof. The cosmetically active agent will typically be present in the composition of the invention in an amount of from about 0.001% to about 20% by weight of the composition, e.g., about 0.01% to about 10% such as about 0.1% to about 5%.

Examples of vitamins include, but are not limited to, vitamin A, vitamin Bs such as vitamin B3, vitamin B5, and vitamin B12, vitamin C, vitamin K, and vitamin E and derivatives thereof.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, glutathiones, resveratrol, lactoferrin, and ascorbic acid and ascorbic acid derivatives (e.g., ascorbyl palmitate, ethyl ascorbic aqcid, ascorbyl glucoside, and ascorbyl polypeptide). Oil-soluble antioxidants suitable for use in the compositions of this invention include, but are not limited to, butylated hydroxytoluene, retinoids (e.g., retinol and retinyl palmitate), tocopherols (e.g., tocopherol acetate), tocotrienols, and ubiquinone. Natural extracts containing antioxidants suitable for use in the compositions of this invention, include, but not limited to, extracts containing flavonoids and isoflavonoids and their derivatives (e.g., genistein and diadzein), extracts containing resveratrol and the like. Examples of such natural extracts include grape seed, green tea, pine bark, and propolis. Other examples of antioxidants may be found on pages 1612-13 of the INCI Handbook.

### Other Materials

various other materials may also be present in the compositions useful in the subject invention. These include humectants, proteins and polypeptides, preservatives and an alkaline agent. Examples of such agents are disclosed in the INCI Handbook, pp.1650-1667. The compositions of the present invention may also comprise chelating agents (e.g., EDTA) and preservatives (e.g., parabens). Examples of suitable preservatives and chelating agents are listed in pp. 1626 and 1654-55 of the INCI Handbook. In addition, the topical compositions useful herein can contain conventional cosmetic adjuvants, such as dyes, opacifiers (e.g., titanium dioxide), pigments, and fragrances.

### Mineral Water

The legume product (e.g., soymilk) and compositions of the present invention may be prepared using a mineral water. In one embodiment, the mineral water has a mineralization of at least about 200 mg/L (e.g., from about 300 mg/L to about 1000 mg/L). In one embodiment, the mineral water comprises at least about 10 mg/L of calcium and/or at least about 5 mg/L of magnesium.

### Use

The present invention also relates to the topical application of legume products or compositions containing such legume products for use in cleansing the skin, hair, or nails. In one embodiment, the present invention is also used in the maintenance of healthy skin, nails, and hair as well as the prevention or the treatment of skin, nails, and hair disorders, including, but not limited to: regulating firmness of the skin, hair, or nails; cleansing the skin, hair or nails; reducing and/or delaying hair or nail growth; straightening and /or lightening of hair; treatment and/or prevention of acne; regulating the tone of skin, hair, or nails; regulating the texture of skin, hair, or nails; regulating wrinkles in skin; treatment of external aggressions in skin, hair, or nails; and beautifying the skin, hair, or nails.

The composition and formulations containing such compositions of the present invention may be prepared using methodology that is well known by an artisan of ordinary skill.

### Example 1: Preparation of Soy Product and Determination Deposition of Salicylic Acid

A formulation was prepared by mixing 2.8%, by weight, of soybean powder (Sunlight Foods Corporation, Taiwan) with 97.2%, by weight, of the cleansing product Clean & Clear® Body Wash (Johnson & Johnson Consumer Products Company, Skillman, New Jersey) which contains 2%, by weight, of the salicylic acid and the lathering surfactants sodium C14-16 olefin sulfate, cocamidopropyl betaine, disodium cocoamphodiacetate, and sodium methyl cocoyl taurate. The mixture was homogenized using a mixer (Cafcamo Stirrer RZR50, Toronto, Ontario, Canada)at high speed and at 25°C for 15 minutes.

The resulting formulation ("Composition 1A") was compared against the cleansing product without the addition of the soybean powder ("Composition 1B") in the following study to determine whether the addition of soybean powder enhanced the amount of salicylic acid deposited to the skin.

First, the arms of each subject were washed with an anti-residue cleanser (Purpose Gentle Cleansing Wash, Johnson & Johnson Consumer Products Company), rinsed for about 1 minute with water, and dried. Then each subject wet the entire surface of his or her volar forearm with 95-100° F water by holding the arm briefly under running tap water. A clinical assistant then marked at least two one-inch diameter circles ("application sites") on the washed volar forearms.

The clinical assistant applied 0.1 ml of either Composition 1A or Composition 1B to each of the application sites. The assistant then wet the index and middle fingers of his/her hand (wearing latex gloves) under running tap water and then moved these fingers in a circular motion over the application site for 10 seconds to lather the product. Lather was retained on the application site for about 90 seconds and then rinsed off under running tap water for 10 seconds. The clinical assistant then gently rubbed with two gloved fingers the site being while it was being rinsed for an additional 5 seconds under tap water.

The steps of the preceding paragraph was then repeated once again at the same application sites, and the subject's arm was pat dried with a paper towel.

Two different measurements were conducted to evaluate the amount of salicylic acid deposited on the each subject's forearm. The first method was the observance of the fluorescence of salicylic acid under an UV lamp. A Black-Ray Lamp (Model UVL-21, UVP Inc., San Gabriel, CA, USA) was operated at long wave UV-366nm, and the UV light was shone on the washed application sites on each of each of the subject's arms. A sensory panel (n=4) was asked to grade the fluoresce intensities emitted from these sites. All of the members of the panel picked the site treated with Composition 1A over the site treated with Composition 1B as having a greater amount of fluorescence.

The second method was the use a fluorometer to read the fluorescene intensity from salicylic acid deposited and, in turn, quantify the salicylic acid value deposited on the treated areas against a USP salicylic acid standard using a spectrofluorimeter (SPEX, Edison, New Jersey, USA). The procedure is set forth in Stamatas et al., J. Invest Dermatol 118:295-302 (2002). The results showed that 15% more salicylic acid was deposited onto the application site from Composition 1A than from Composition 1B (n=5). The fluorescence measurement error is less than 5% (RSD).

### Example 2: Comparison of Increasing Amount of Legume Product on Deposition of Salicylic Acid

The following study was conducted to determine the effect of increasing the amount of legume product on the deposition of salicylic acid from a cleansing product. Compositions containing various amounts of soybean powder and Cleans .& Clear® Body Wash were manufactured as described above in Example 2. Relative salicylic acid fluorescence intensity, as described in Example 2, was used to measure the level of salicylic acid deposition. As depicted in Table I, a dose dependent effect for salicylic acid deposition was found by increasing the amount of soybean powder from 1% to 5%, by weight. The product containing 5% soybean powder demonstrated a 36% increase in salicylic acid fluorescence as compared to the same product containing only 1% soybean powder.

**Table 2**

| Soy Concentration (wt/wt %) | Percent Increase of Salicylic Acid Fluorescence Intensity |
|---|---|
| 1% | N/A |
| 2% | 19% |
| 5% | 36% |

It is understood that while the invention has been described in conjunction with the detailed description thereof, that the foregoing description is intended to illustrate and not limit the scope of the invention, which is defined by the scope of the appended claims.

## Claims

1. A cleansing composition comprising (i) a surfactant, (ii) a legume product selected from the group consisting of soybean powder, soymilk powder, and mixtures thereof, and (iii) a cosmetically active organic acid or a cosmetically acceptable salt or ester thereof.

2. A composition of claim 1, wherein said surfactant is a lathering surfactant.

3. A composition of claim 1, wherein said cosmetically active organic acid is a hydroxy acid.

4. A composition of claim 2, wherein said cosmetically active organic acid is a hydroxy acid.

5. A composition of claim 1, wherein said cosmetically active organic acid is salicylic acid.

6. A composition of claim 2, wherein said cosmetically active organic acid is salicylic acid.

7. A composition of claim 1, wherein said composition comprises from about 0.1 to about 20 percent, by weight, of said legume product.

8. A composition of claim 2, wherein said composition comprises from about 0.1 to about 20 percent, by weight, of said legume product.

9. A composition of claim 3, wherein said composition comprises from about 0.1 to about 20 percent, by weight, of said legume product.

10. A composition of claim 5, wherein said composition comprises from about 0.1 to about 20 percent, by weight, of said legume product.

11. A method of cleansing the skin, hair, or nail, said method comprising:
(a) applying to the skin, hair, or nail a cleansing composition comprising (i) a surfactant, (ii) a legume product selected from the group consisting of soybean powder, soymilk powder, and mixtures thereof, and (iii) a cosmetically active organic acid or a cosmetically acceptable salt or ester thereof, and
(b) removing said composition from said skin, hair, or nail.

12. A method of claim 11, wherein said surfactant is a lathering surfactant.

13. A method of claim 11, wherein said cosmetically active organic acid is a hydroxy acid.

14. A method of claim 12, wherein said cosmetically active organic acid is a hydroxy acid.

15. A method of claim 11, wherein said cosmetically active organic acid is salicylic acid.

16. A method of claim 12, wherein said cosmetically active organic acid is salicylic acid.

17. A method of claim 11, wherein said composition comprises from about 0.1 to about 20 percent, by weight, of said legume product.

18. A method of claim 12, wherein said composition comprises from about 0.1 to about 20 percent, by weight, of said legume product.

19. A method of claim 13, wherein said composition comprises from about 0.1 to about 20 percent, by weight, of said legume product.

20. A method of claim 15, wherein said composition comprises from about 0.1 to about 20 percent, by weight, of said legume product.

21. A method of enhancing the deposition of a cosmetically active organic acid or a cosmetically acceptable salt or ester thereof from a cleansing composition onto the skin, said method comprising adding a legume product to said composition, wherein said composition comprises a surfactant.

## Patentansprüche

1. Reinigungs-Zusammensetzung, umfassend (i) einen grenzflächenaktiven Stoff, (ii) ein Gemüseprodukt, ausgewählt aus der Gruppe, bestehend aus Sojabohnenpulver, Sojamilchpulver und Mischungen derselben, und (iii) eine kosmetisch-aktive organische Säure oder ein kosmetisch annehmbares Salz oder Ester derselben.

2. Zusammensetzung nach Anspruch 1, **dadurch gekennzeichnet, daß** der grenzflächenaktive Stoff ein schaumfähiger grenzflächenaktiver Stoff ist.

3. Zusammensetzung nach Anspruch 1, **dadurch gekennzeichnet, daß** die kosmetisch-aktive organische Säure eine Hydroxysäure ist.

4. Zusammensetzung nach Anspruch 2, **dadurch gekennzeichnet, daß** die kosmetisch-aktive organische Säure eine Hydroxysäure ist.

5. Zusammensetzung nach Anspruch 1, **dadurch gekennzeichnet, daß** die kosmetisch-aktive organische Säure Salicylsäure ist.

6. Zusammensetzung nach Anspruch 2, **dadurch gekennzeichnet, daß** die kosmetisch-aktive organische Säure Salicylsäure ist.

7. Zusammensetzung nach Anspruch 1, **dadurch gekennzeichnet, daß** die Zusammensetzung einen Massenanteil von zirka 0,1 bis zirka 20 Prozent des Gemüseprodukts umfaßt.

8. Zusammensetzung nach Anspruch 2, **dadurch gekennzeichnet, daß** die Zusammensetzung einen Massenanteil von zirka 0,1 bis zirka 20 Prozent des Gemüseprodukts umfaßt.

9. Zusammensetzung nach Anspruch 3, **dadurch gekennzeichnet, daß** die Zusammensetzung einen Massenanteil von zirka 0,1 bis zirka 20 Prozent des Gemüseprodukts umfaßt.

10. Zusammensetzung nach Anspruch 5, **dadurch gekennzeichnet, daß** die Zusammensetzung einen Massenanteil von zirka 0,1 bis zirka 20 Prozent des Gemüseprodukts umfaßt.

11. Verfahren zum Reinigen der Haut, des Haares oder der Nägel, wobei dieses Verfahren umfaßt:
(a) Aufbringen einer Reinigungszusammensetzung auf die Haut, das Haar oder die Nägel, umfassend: (i) einen grenzflächenaktiven Stoff, (ii) ein Gemüseprodukt, ausgewählt aus der Gruppe, bestehend aus Sojabohnenpulver, Sojamilchpulver und Mischungen derselben, und (iii) eine kosmetisch-aktive organische Säure oder ein kosmetisch annehmbares Salz oder Ester derselben, und
(b) Entfernen der Zusammensetzung von der Haut, aus dem Haar oder von den Nägeln.

12. Verfahren nach Anspruch 11, **dadurch gekennzeichnet, daß** der grenzflächenaktive Stoff ein schaumfähiger grenzflächenaktiver Stoff ist.

13. Verfahren nach Anspruch 11, **dadurch gekennzeichnet, daß** die kosmetisch-aktive organische Säure eine Hydroxysäure ist.

14. Verfahren nach Anspruch 12, **dadurch gekennzeichnet, daß** die kosmetisch-aktive organische Säure eine Hydroxysäure ist.

15. Verfahren nach Anspruch 11, **dadurch gekennzeichnet, daß** die kosmetisch-aktive organische Säure Salicylsäure ist.

16. Verfahren nach Anspruch 12, **dadurch gekennzeichnet, daß** die kosmetisch-aktive organische Säure Salicylsäure ist.

17. Verfahren nach Anspruch 11, **dadurch gekennzeichnet, daß** die Zusammensetzung einen Massenanteil von zirka 0,1 bis zirka 20 Prozent des Gemüseprodukts umfaßt.

18. Verfahren nach Anspruch 12, **dadurch gekennzeichnet, daß** die Zusammensetzung einen Massenanteil von zirka 0,1 bis zirka 20 Prozent des Gemüseprodukts umfaßt.

19. Verfahren nach Anspruch 13, **dadurch gekennzeichnet, daß** die Zusammensetzung einen Massenanteil von zirka 0,1 bis zirka 20 Prozent des Gemüseprodukts umfaßt.

20. Verfahren nach Anspruch 15, **dadurch gekennzeichnet, daß** die Zusammensetzung einen Massenanteil von zirka 0,1 bis zirka 20 Prozent des Gemüseprodukts umfaßt.

21. Verfahren für die Förderung der stärkeren Ablagerung einer kosmetischaktiven organischen Säure oder eines kosmetisch annehmbaren Salzes oder Esters derselben aus einer Reinigungs-Zusammensetzung auf der Haut, wobei das Verfahren die Hinzufügung eines Gemüseprodukts zur Zusammensetzung umfaßt, **dadurch gekennzeichnet, daß** die Zusammensetzung einen grenzflächenaktiven Stoff umfaßt.

## Revendications

1. Composition de nettoyage comprenant (i) un agent tensioactif, (ii) un produit végétal choisi dans le groupe constitué par une poudre de soja, une poudre de lait de soja et des mélanges de celles-ci, et (iii) un acide organique actif d'un point de vue cosmétique ou un sel ou un ester de celui-ci acceptable d'un point de vue cosmétique.

2. Composition selon la revendication 1, dans laquelle ledit agent tensioactif est un agent tensioactif moussant.

3. Composition selon la revendication 1, dans laquelle ledit acide organique actif d'un point de vue cosmétique est un hydroxyacide.

4. Composition selon la revendication 2, dans laquelle ledit acide organique actif d'un point de vue cosmétique est un hydroxyacide.

5. Composition selon la revendication 1, dans laquelle ledit acide organique actif d'un point de vue cosmétique est l'acide salicylique.

6. Composition selon la revendication 2, dans laquelle ledit acide organique actif d'un point de vue cosmétique est l'acide salicylique.

7. Composition selon la revendication 1, dans laquelle ladite composition comprend d'environ 0,1 à environ 20 % en poids dudit produit végétal.

8. Composition selon la revendication 2, dans laquelle ladite composition comprend d'environ 0,1 à environ 20 % en poids dudit produit végétal.

9. Composition selon la revendication 3, dans laquelle ladite composition comprend d'environ 0,1 à environ 20 % en poids dudit produit végétal.

10. Composition selon la revendication 5, dans lequelle ladite composition comprend d'environ 0,1 à environ 20 % en poids dudit produit végétal.

11. Procédé de nettoyage de la peau, des cheveux ou des ongles, ledit procédé comprenant :
(a) l'application sur la peau, les cheveux ou les ongles d'une composition de nettoyage comprenant (i) un agent tensioactif, (ii) un produit végétal choisi dans le groupe constitué par une poudre de soja, une poudre de lait de soja et des mélanges de celles-ci, et (iii) un acide organique actif d'un point de vue cosmétique ou un sel ou un ester de celui-ci acceptable d'un point de vue cosmétique, et
(b) le retrait de ladite composition de ladite peau, desdits cheveux ou desdits ongles.

12. Procédé selon la revendication 11, dans lequel ledit agent tensioactif est un agent tensioactif moussant.

13. Procédé selon la revendication 11, dans lequel ledit acide organique actif d'un point de vue cosmétique est un hydroxyacide.

14. Procédé selon la revendication 12, dans lequel ledit acide organique actif d'un point de vue cosmétique est un hydroxyacide.

15. Procédé selon la revendication 11, dans lequel ledit acide organique actif d'un point de vue cosmétique est l'acide salicylique.

16. Procédé selon la revendication 12, dans lequel ledit acide organique actif d'un point de vue cosmétique est l'acide salicylique.

17. Procédé selon la revendication 11, dans lequel ladite composition comprend d'environ 0,1 à environ 20 % en poids dudit produit végétal.

18. Procédé selon la revendication 12, dans lequel ladite composition comprend d'environ 0,1 à environ 20 % en poids dudit produit végétal.

19. Procédé selon la revendication 13, dans lequel ladite composition comprend d'environ 0,1 à environ 20 % en poids dudit produit végétal.

20. Procédé selon la revendication 15, dans lequel ladite composition comprend d'environ 0,1 à environ 20 % en poids dudit produit végétal.

21. Procédé de stimulation du dépôt d'un acide organique actif d'un point de vue cosmétique ou d'un sel ou d'un ester de celui-ci acceptable d'un point de vue cosmétique provenant d'une composition de nettoyage sur la peau, ledit procédé comprenant l'ajout d'un produit végétal à ladite composition, dans lequel ladite composition comprend un agent tensioactif.
